# EUROPEAN PATENT APPLICATION

(11) **EP 2 036 497 A1**
(43) Date of publication of application: **18.03.2009**
(21) Application number: 07116539.3
(22) Date of filing: 17.09.2007
(51) Int. Cl.: A61B 6/00, A61B 8/06, A61B 5/055

(54) **Method for generating quantitative images of the flow potential of a region under investigation**

(71) Applicant: Amid s.r.l., 67039 Sulmona (AQ) (IT)
(72) Inventor: Pedrizzetti, Gianni, I-59100, Prato (IT); Tonti, Giovanni, I-67039, Sulmona (AQ) (IT); Marchese, Emidio, I-65026, Popoli (PE) (IT)

(57) **Abstract**

The invention relates to a method for generating images representatives of the flow potential of a permeable body comprising the following steps:
- providing a first sequence of digital images of the body perfused by a fluid at a rest condition;
- extracting and/or calculating at least one first quantification image of the spatial distribution of flow of said fluid in said body at said rest condition, said at least one first quantification image being defined by pixels or group of pixels values having an appearance scale univocally correlated to a parameter representative of the perfusion at said rest condition;
- providing a second sequence of digital images of the body perfused by the fluid at a stress condition, wherein at such stress condition the perfusion is forced to be increased with respect to the rest condition;
- extracting and/or calculating at least one second quantification image of the spatial distribution of flow of said fluid in said body at said stress condition, said at least one second quantification image being defined by pixels or group of pixels values having an appearance scale univocally correlated to a parameter representative of the perfusion at said stress condition;

- combining the two quantification images to obtain at least an image defined by pixels or group of pixels values having an appearance scale univocally correlated to a combination of corresponding pixels or group of pixels of the two quantification images.

## Description

The present invention relates to a method for generating images of a region under investigation where the image information is related to the capability of such region to allow the flow of a fluid.

It is generally known that the capability of non-rigid pipes or conduits to allow a fluid to pass through can be determined by measuring the flow when the fluid is in a steady state condition and during transient state when the fluid is forced to flow at an increased rate. The differential value thus obtained is an indirect evaluation of the capability of the conduits to deform to comply with an increased demand of fluid flow. This differential approach has however several major drawbacks which are mainly due to the fact that it consists of single local measurements that are not informative of the spatial distribution of flow in a region under analysis, but only of the capability (reserve) that single conduits have to increase the flow. When structures having a large number of small conduits or permeable bodies have to be analysed, such methodology requires a distinct differential analysis for each of the flow patterns that can be identified which is a rather complex if not impracticable task for very complex structures.

In the medical field this approach is used in the so-called Coronary Flow Reserve (CFR) where the ability of coronary vessels to increase the blood flow during a stress test is measured.

Coronary arteries are vessels that transport oxygenated blood and nutrients to the heart supplying so the substrates required for the myocardial contraction. When a coronary artery has a critical obstruction it becomes unable to deliver the proper amount of oxygen and nutrients to the interested region causing ischemia. A complete coronary obstruction is the pathological basis of myocardial infarction.

Even relevant coronary obstruction (generally up to 75%) may be asymptomatic at rest and sometimes are difficult to detect in the clinical practice. In such clinical conditions, an increase of myocardial oxygen request, exceeding a particular threshold (Coronary Flow Reserve), e.g. during physical exercise or other stressing condition, may give rise to myocardial ischemia or necrosis.

CFR is usually calculated as the ratio between maximal (stress, hyperemic) to resting coronary blood flow. Stress is obtained by injection of a vasodilator drug such as adenosine or dypiridamole or by physical exercise to achieve the maximal dilation of microcirculation. Coronary flow velocities, at rest and peak of stress, are measured by means of pulsed Doppler technique directly on the coronary vessel visualized during an echocardiographic exam. The ratio between the value of flow velocity at stress and at rest is the CFR. For details see for example Dimitrow PP, Galderisi M, Rigo F. The non-invasive documentation of coronary microcirculation impairment: role of transthoracic echocardiography. Cardiovascular Ultrasound, 2005; 3:18-26.

The diagnosis of non-limiting flow coronary lesion is still a challenge in the clinical practice. In most instances it is based on the anatomical measurement of the effective degree of stenosis by means of invasive methods (like angiography or intravascular ultrasound). The invasive methods are still quite expensive, are available only in largest medical centers and, even now, imply a little but actual risk of serious complications. Non invasive methods of nuclear medicine (like SPECT or PET) are expensive too, require the administration of radioactive isotopes and have a limited repeatability. To further complicate the question, other ischemic conditions are associated with a damage of the smaller myocardial vessels (microcirculation) sometimes without any evidence of critical stenosis of the large epicardial coronaries (X syndrome). In these cases, being unable to visualize the microcirculation, also the invasive methods fail to detect the pathological condition.

CFR is an important functional parameter to understand the pathophysiology of coronary circulation especially because it can be evaluated in a non-invasive manner without requiring catheterisation as, for example, in angiography and more in general in the methods disclosed in Tobis J, Azarbal B, Slavin L. Assessment of Intermediate Severity Coronary Lesions in the Catheterization Laboratory. J Am Coll Cardiol 2007; 49:839-848.

However CFR, being a local differential analysis, cannot determine a real estimate of the actual flow of the microcirculation of the myocardium. CFR is, in fact, only a measure of the global capacity of flow in the coronaries well upstream the myocardial region. This results in a non accurate evaluation of a necrosis due for example to stroke. In fact it may happen in the so-called no-reflow status that a stenosis in a coronary artery determines a necrosis of the micro-circulation in a zone of the myocardium. Such stenosis causes a global reduction of the capacity of flow of the coronary which can be identified by CFR. However, once such stenosis is removed, for example by using a stent or with angioplasty, the flow in the diseased artery becomes normal again and CFR cannot help evaluate if the micro-circulation is irremediably compromised and if such zone of the myocardium is no longer able to react to an increased demand for blood flow under stress as no information of the spatial distribution of blood flow in the myocardial microcirculation can be determined with CFR. Furthermore not all the coronary vessels are easily visible during a standard echo examination and not in all patients is possible to see the coronary flow with colour Doppler thus limiting the application of CFR analysis only to those coronaries that can be reached in a trans-thoracic projection, namely the left anterior descending coronary artery and the right coronary artery

It is thus an object of the present invention to provide for a method for determining in an easy and effective way the capability of a body or an area to respond to a variation of flow demand.

Perfusion, that corresponds to the physical phenomenon commonly called filtration, represents the flow of a fluid into a porous or permeable medium. An example may be the diffusion of a polluting agent through the soil or more in general the passage of a fluid through a filtering medium also called percolation or infiltration. In the medical and/or veterinary field the term perfusion is generally used to identify the blood flow into a micro vascular tree.

In cardiology practice, the ability to evaluate, in a quantitative way, the myocardial perfusion, is of primary importance. In other branches of medicine perfusion is measured in kidney, liver, or other parenchymal organs to assess proper perfusion, lack of perfusion (necrotic region), or anomalous pattern of perfusion (cancer). For a reference see, for example, Becher H., Burns PN. Handbook of Contrast Echocardiography 2000 Springer-Verlag. ISBN 3-540-67083, Gibson CM, Cannon CP, Murphy SA, Marble SJ, Barron HV, Braunwald E. Relationship of the TIMI Myocardial Perfusion Grades, Flow Grades, Frame Count, and Percutaneous Coronary Intervention to Long-Term Outcomes After Thrombolytic Administration in Acute Myocardial Infarction. Circulation 2002; 105:1909-1913, Blomley MJK, Dawson P. Bolus dynamics: theoretical and experimental aspects. The British Journal of Radiology 1997; 70:351-359, Rausch M, Scheffler K, Rudin M, Radu EW. Analysis of input functions from different arterial branches with gamma variate functions and cluster analysis for quantitative blood volume measurements. Magnetic Resonance Imaging 2000; 18:1235-1243.

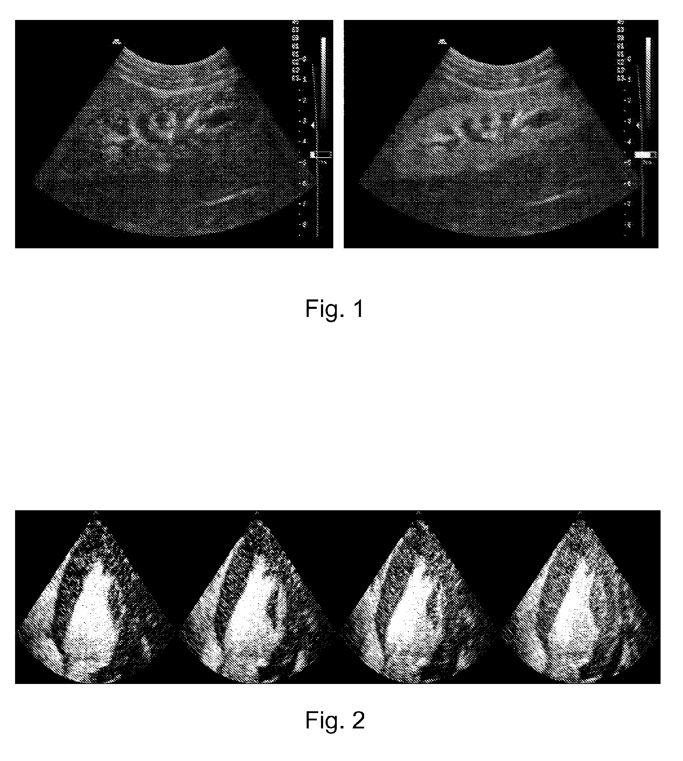

Perfusion is visualized in medical diagnostic equipments based on advanced imaging techniques, in particular Echography, MRI, Angiography, PET, SPECT; images are generally obtained after infusion of a contrast agent that is created to be particularly well visible in the specific imaging modality. Such a contrast agent is a marker for blood and therefore permits to visualize the tissue uptake of blood while imaging as an increase of the brightness of the tissue, as shown in figure 1 and 2 where ultrasound perfusion images of the kidney and the left ventricle are respectively illustrated. The maximum brightness in a region of tissue is a measure of cross-sectional area of the viable vessels in such region; the rapidity with which such tissue increase its brightness is a measure of the flow of blood. For further details see, for example, Wei K, Jayaweera AR, Firoozan S, Linka A, Skyba DM, Kaul S. Quantification of Myocardial Blood Flow With Ultrasound-Induced Destruction of Microbubbles Administered as a Constant Venous Infusion. Circulation 1998; 97:473-483.

This approach has however several major drawbacks as the perfusion is a measure of brightness and not of blood flow. Although brightness is related to the amount of contrast agent and therefore of blood, this relation is, in fact, not absolute and depends on several settings that are not controllable and/or normally not reproducible with confidence. Furthermore no information can be derivable on the capacity of a perfused object or organ to react to a demand of an increased fluid flow.

It is thus another object of the present invention to provide for a method for determining accurate and reproducible spatial perfusion measurements.

The applicant has now observed that a synergic combination of differential flow analysis and perfusion imaging can surprisingly contribute to solve the problems associated to both. In fact the use of perfusion imaging to measure flow properties allows to focus directly on microcirculation, and to evidence the spatial distribution of microcirculation flow thus solving the main drawbacks of differential flow analysis in general and CFR in particular. On the other hand, the application of the flow reserve concept to the perfusion measures implies that a microcirculation flow of an area is evaluated, during stress, relatively to the same area at rest. A target body is thus used as reference for itself. In medical applications that means that a patient is used as control for her/himself thus solving the main drawback of perfusion imaging.

The invention reaches the aims with a method for generating images representatives of the flow potential of a permeable body comprising the following steps:
- providing a first sequence of digital images of the body perfused by a fluid at a rest condition;
- extracting and/or calculating at least one first quantification image of the spatial distribution of flow of said fluid in said body at said rest condition, said at least one first quantification image being defined by pixels or group of pixels values having an appearance scale univocally correlated to a parameter representative of the perfusion at said rest condition;
- providing a second sequence of digital images of the body perfused by the fluid at a stress condition, wherein at such stress condition the perfusion is forced to be increased with respect to the rest condition;
- extracting and/or calculating at least one second quantification image of the spatial distribution of flow of said fluid in said body at said stress condition, said at least one second quantification image being defined by pixels or group of pixels values having an appearance scale univocally correlated to a parameter representative of the perfusion at said stress condition;
- combining the two quantification images to obtain at least an image defined by pixels or group of pixels values having an appearance scale univocally correlated to a combination of corresponding pixels or group of pixels of the two quantification images.

Thanks to the method according to the invention it is thus possible to build, from at least two sequences of digital images, such as for example echographic loops, an image having pixels or group of pixels arranged in a pixel array in the correct or approximately correct spatial relation to the other pixels or group of pixels as their spatial relation existing in the real object and with a value representative of one or more parameters related to the flow potential of corresponding points or area of the real object. In this way an immediate grasp of a zone with poor flow capability (reserve) can be determined, especially if the resulting image is superimposed on an image of the body under investigation, for example by advantageously varying the opacity of such image as taught in the international application published with the number WO2005/054898.

Quantification images of the spatial distribution of flow can be determined, for example, by following the teachings of the European patent application published with number EP-A-1519315 or any other known method which allows to calculate images representative of the perfusion.

The at least two quantification images are typically aligned so that pixel values of one image can be compared to homologous pixel values of the other image, for example by deforming one or both images to allow cross reference points identified on both images to overlap. Such reference points could be landmarks, i.e. representative points or segments identified on each image, like, for example, in medical applications, easily discernible anatomic features. In cardiology images, such landmarks could be, for example, the two extremities of the annulus and the cardiac apex. Alternatively or in combination the images can be aligned manually or automatically using optimal-likelihood-based processing.

Advantageously the at least two quantification images are combined through a non-linear software and/or hardware device, such as a divider and/or a multiplier and/or a logarithmic and/or a cross-correlation circuit or the like.

Preferably the step of combining the two quantification images comprises calculating the ratio of pixel values of the second image to corresponding pixel values of the first image or vice versa to obtain an image where some or all the pixels have values corresponding to such ratio. One or more thresholds may be defined to determine which pixels of the generated image(s) are a combination of the corresponding pixels of the first and second image and/or which pixels of the generated image(s) correspond to pixels related to only one of the two images and/or are filtered out. For example by neglecting and/or correcting values below such threshold(s) noise can be filtered improving the quality of the generated image.

The step of extracting and/or calculating at least one quantification image from each sequence of images typically comprises one or more steps selected from the group consisting of:
- extracting the last frame of the sequence;
- extracting a frame of the sequence at an intermediate time;
- extracting/calculating the image of the sequence having the maximum brightness;
- determining a parametric image.
Particularly the step of determining a parametric image comprises:
- defining an evaluation function for each sequence of images, said evaluation function having at least one parameter;
- calculating for each pixel or group of pixels of each sequence the value of said parameter for best fitting said evaluation function with a curve representing the values of said pixels or group of pixels obtained from such sequence of images;
- constructing a parametric image for each sequence of images by defining a pixel appearance scale univocally correlated to said at least one parameter.

The estimation function is typically of the form y(t)=A(1-exp(-Bt)), where y(t) is the pixel value depending from time, t is the time at which the pixel value has been determined in the image and A and B are parameters giving the best fit of said estimation function.

The parameters may be imaged in a two-dimensional or a three-dimensional image, or in any known representation of a function of one, two, or more variables with pixel values comprising the brightness of black and white digital images or one or more variables of colour digital images like hue, saturation, colour or the like.

According to an embodiment, more quantification images are extracted and/or calculated from each sequence of images, more images being generated by combining corresponding images at rest and stress condition. Alternatively or in combination only one image is generated, such image being the ratio of two images extracted/calculated from a combination of images extracted and/or calculated respectively from the sequence of images at stress and from the sequence of images at rest.

Advantageously the generated image(s) is/are displayed overlaid with the image(s) of the first and/or the second sequence of images and/or with the corresponding quantification image(s), for example by varying the opacity of such images.

The images of the sequences may echographic or MRI or SPECT or PET or X-Ray images or the like and may be, for example, obtained with one or more imaging modes selected from the group consisting of: Doppler, power Doppler, B-mode, Harmonic imaging, Contrast imaging.

According to an embodiment the permeable body is a biological tissue, the sequences of images being representative of the spatial distribution of blood flow in such tissue. Particularly the permeable body is the heart, the sequences of images being representative of the blood perfusion in the myocardium, for example images having brightness values related to the concentration of a contrast media perfusing the myocardium at the time the images are taken.

Advantageously for each sequence of images representative of a condition of the heart, a perfusion image is determined, such perfusion image giving a synthetic spatial representation of the perfusion process of at least part of the myocardium at such condition. One or more differential perfusion images representative of the capacity of the myocardium to react to a demand for an increased blood flow may be determined by combining perfusion images obtained from sequences of images of the myocardium at rest and hyperemic condition.

Further improvements of the invention will form the subject of the dependent claims.

The characteristics of the invention and the advantages derived therefrom will be more apparent from the following description of non-limiting embodiments, illustrated in the annexed drawings, in which:
Fig. 1 shows ultrasound images of kidney during perfusion. Left: initial stage with little contrast agent. Right: the organ is saturated of contrast agent and the brightness is a measure of the viable vessels. The time to reach saturation is a measure of the velocity of blood.
Fig. 2 shows a sequence of ultrasound images of perfusion in the left ventricle. The blood pools are bright from the beginning, the myocardium is darker. From left to right: the myocardium is initially black and get more and more filled with contrast agent (brighter). The maximum brightness is a measure of the volume of viable vessels; the rapidity of brightness increase is a measure of the flow of blood.

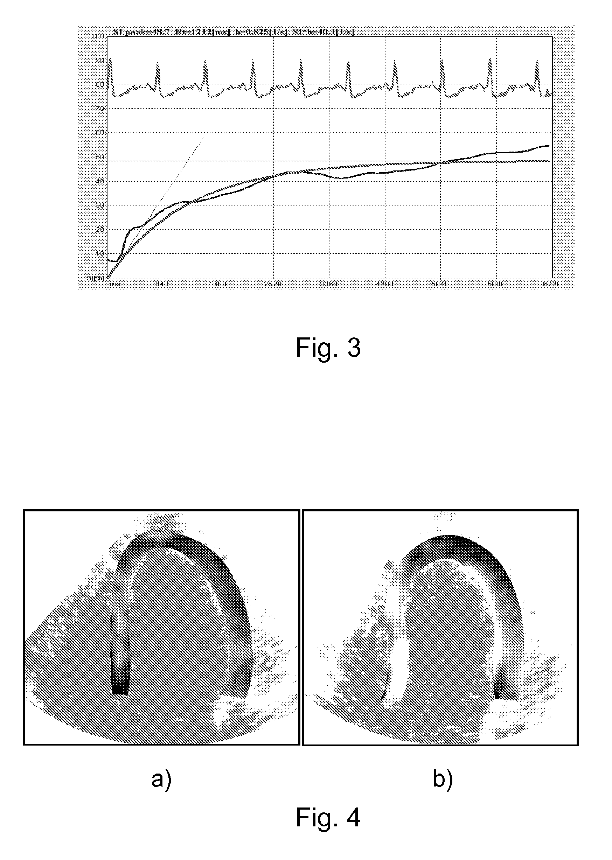

Fig. 3 shows the time profile of signal intensity (brightness), SI(t), is a representation of the local perfusion process. A parametric curve, in this case an exponential curve SI(t)=A(1-exp(-Bt)), with two parameters A and B, can be adapted to the perfusion curve and the value of the parameters are a synthetic quantitative measure of the perfusion process, for example blood flow.
Fig. 4 shows parametric images of microcirculation evaluated from imaging of perfusion in the myocardium. Results for a same patient at rest state (left) and stress (right) are shown. The quantitative part of the images covers the myocardium, in both images the quantified region is delimitated by the two curves corresponding to endocardium and epicardium and by the annulus on left and right sides of the mitral valve. An increase of perfusion flow, in term of brightness, is noticeable in several segments on the image recorded at stress conditions (b), with respect to basal state (a).

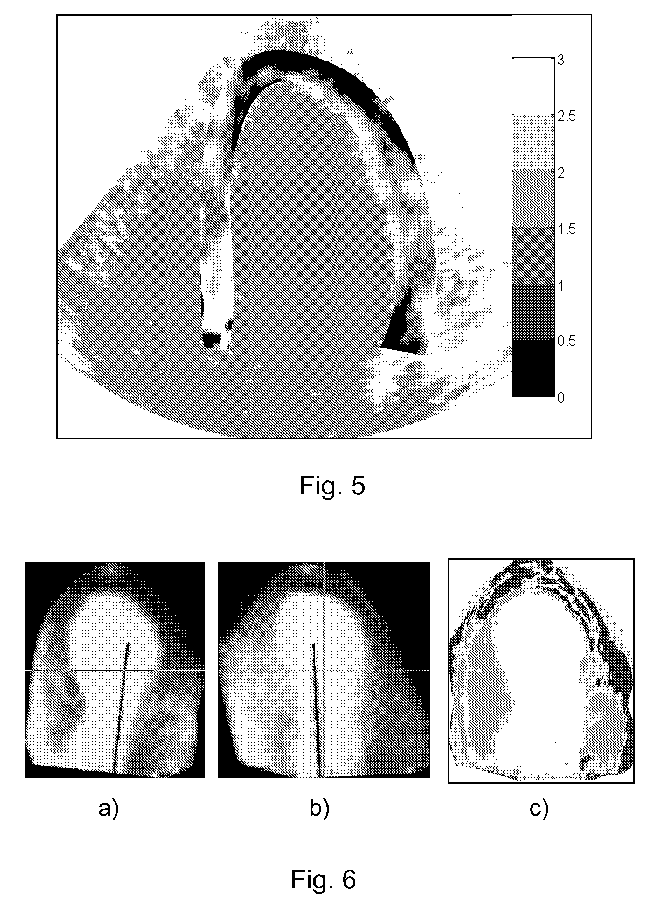

Fig. 5 shows an image of the microcirculation reserve in the myocardium according to an embodiment of the invention. The two parametric images of figure 4, that were mathematically equivalent to rectangles, where overlapped and then the ratio of the stress with respect to baseline is shown, the resulting image shows the increase of flow that occurs during stress.
Fig. 6 shows an image of the microcirculation reserve in the myocardium (right) according to a second embodiment of the invention. The image (c) is computed as the ratio between the rest (a) and stress (b) perfusion images recorded at saturation. Overlapping was here considered accurate without deformations. The two darker colour levels in the resulting image (c) correspond to value above and below the value of 1.5.

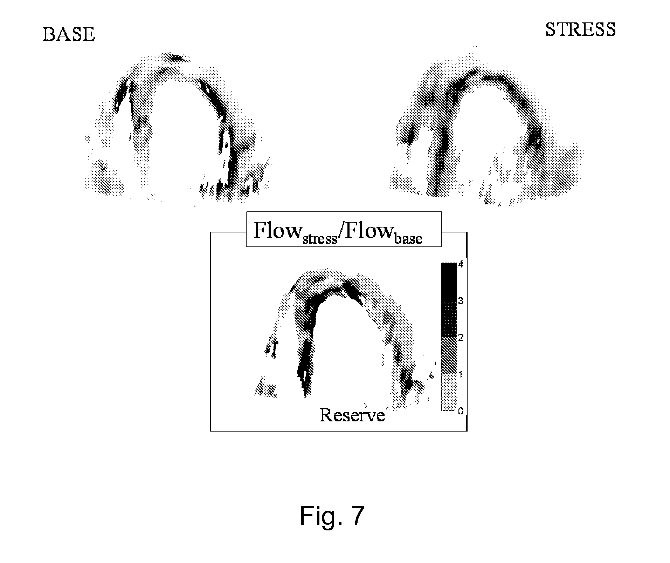

Fig. 7 shows an image of the microcirculation reserve in the myocardium (below) according to a third embodiment of the invention. The image is computed from parametric imaging of flow at rest and stress (above) computed from perfusion clips. Alignment of the two parametric images was obtained by deforming the stress image to have an overlapping of its myocardium median line onto the same line of the baseline image.

The invention will be now described with reference to bi-dimensional ultrasound images of organs, particularly the heart, however the skilled person would appreciate that the inventive concept can be applied to process any kind of sequence of images of bodies or regions perfused by a fluid from which quantification images of the spatial distribution of flow can be determined.

With reference to Fig. 1 and 2, a sequence of ultrasound images of perfusion in the kidney and in the left ventricle are respectively shown. From left to right the organ is gradually filled with contrast agent till a saturation is reached. The maximum brightness of the pixels is a measure of the viable vessels in the two organs, while the time to reach saturation is a measure of the velocity of blood. A simple method to estimate the maximum brightness and the speed of brightness increase is from the acquired images directly, typically from the images at a final and intermediate times. More quantitatively accurate estimates are obtained by drawing, at a selected region, a curve of brightness as a function of time, then by adapting a parametric curve as those depicted in Fig. 3 to the perfusion curve, and obtain the value of the corresponding parameters. Such parameters are measures of specific properties of the microcirculation flow. In more advanced approaches, such parametric adaptation is performed at all or many places in the tissue such that images of the parameters can be obtained. These, so called parametric images, are synthetic representation of the perfusion process for the entire tissue; these give an imaging that quantitatively describe the microcirculation flow in the organ. For a reference see, for example, Agati L, Tonti G, Pedrizzetti G, Magri F, Funaro S, Madonna M, Celani F, Messager T, Broillet A. Clinical application of quantitative analysis in real-time MCE. Eur J Echocardiography 2004; 45:S9-S15. Agati L, Tonti G, Pedrizzetti G. Clinical Application of Quantitative Analysis in Myocardial Contrast Echocardiography. In: Contrast Echocardiography in Clinical Practice, JL Zamorano, MA Garcia-Fernandez eds. Springer 2004. ISBN 88-470-0237-0.

The method according to the invention is essentially based on the processing of at least a pair of perfusion clips, or image sequences, recorded from the same object or area at rest (clip A) and during stress (clip B), respectively. The first clip is used as the baseline, the second clip is thus used to evaluate the flow increase relatively to that baseline.

The processing can be summarized with the following exemplary steps:

### CLIP QUANTIFICATION INTO PARAMETRIC IMAGES:

- Analyze digital clip A and extract one image, say image A, that is a quantification of the flow in imaged tissue. Examples of such images can be the last frame of the sequence (final perfusion, figure 6), or one frame at an intermediate time, or the image of the maximum brightness. More advanced examples are parametric images of the perfusion process (figure 4, 7). Parametric images would be the preferred choice, for example determined according to the teachings of EP-A-1519315.
- Analyze digital clip B in the same way of clip A. Obviously step A and B can also be performed in reverse order.

### ALIGNMENT OF PARAMETRIC IMAGES:

- Align the two obtained quantitative images, image A and image B, in such a way that values at a certain position in the image A can be compared with values in a homologous position in the image B. This can be performed by deforming one or both images in a way that points corresponding to physiological elements (or other reference) in one image overlap with the same elements in the other image. For example the parametric imaging of the myocardium can be deformed, see figure 4, by aligning the endocardium and the epicardium and the annulus; or by aligning the myocardial center (figure 7). Alignment can be performed manually or, preferably, automatically on the basis of the information available or with other optimal likelihood based processing.

### COMPARISON OF ALIGNED IMAGES:

- Perform the ratio of values contained in image B to values in the same position, after alignment, of image A, and obtain one new image where every element contains such ratio. For best result, such a ratio should avoid divisions between values that are not significant. This ratio is an image which can be conveniently called hereinafter Perfusion Flow Reserve Image or Micro-Circulation Reserve Image.

### These steps are now described with the aid of pictures.

Example results after the first processing step are shown in figure 4 where two clips of myocardial perfusion in a same patient at rest (a) and during stress (b) are quantified. The pictures report the parametric images of myocardial perfusion flow that cover the myocardium, these are plotted on top of one frame of the perfusion clip for easier interpretation. The basal perfusion image (a) shows an approximately uniformly perfusion, however its comparison with the stress perfusion image (b) shows a substantial increase of perfusion on the side walls of the ventricle (septum on the left, and lateral wall on right) while the apical part of the tissue does not presents the increase that should be expected during stress. The stress exam thus evidences, qualitatively, a critical state for this patient whose coronary vessels upstream the apical muscular segments present a viability problem. Both parametric images are mathematically equivalent to rectangular images that extend over a band ranging from endocardium to epicardium, and from one side to the other side of the annulus. Overlapping of the second image on the first one is thus simply achieved by stretching the two dimensions of the rectangles to have these borders aligned. The ratio between the values of the two images is reported on figure 5, this Micro-circulation Flow Reserve Image is plotted on top of one frame of the first perfusion clip for clarity. The image on figure 5 now gives a quantitative information on the degree of insufficiency on coronary flow reserve. Quantitative information allows to evaluate objectively the influence of a therapy on reflowing or presence of partial necrosis, either at an acute state after an urgent treatment or during recovery and follow-up.

Figure 6 shows the same process performed on the saturation images (maximum brightness) of perfusion recording at baseline (a) and during stress (b). In this case the alignment of images could be considered superfluous and is not performed, the microcirculation reserve image (c) shows region where perfusion is increased under stress by a factor above 1.5, and regions where increase is below 1.5, indicating presence of pathological phenomenon. These, here shown in darker gray, extend over a wide part of the apex up to the middle lateral wall.

Figure 7 shows the same process performed on parametric images of flow determined, for example, according to the teachings of the already mentioned papers by Agati et. al. or EP-A-1519315, and reserve is computed after a simple alignment of the median myocardial line.

Although the method according to the invention has been mainly described with reference to bi-dimensional images, it can be also used with three-dimensional perfusion imaging all without departing from the guiding principle of the invention disclosed above and claimed below.

## Claims

1. Method for generating images representatives of the flow potential of a permeable body **characterised in** comprising the following steps:
- providing a first sequence of digital images of the body perfused by a fluid at a rest condition;
- extracting and/or calculating at least one first quantification image of the spatial distribution of flow of said fluid in said body at said rest condition, said at least one first quantification image being defined by pixels or group of pixels values having an appearance scale univocally correlated to a parameter representative of the perfusion at said rest condition;
- providing a second sequence of digital images of the body perfused by the fluid at a stress condition, wherein at such stress condition the perfusion is forced to be increased with respect to the rest condition;
- extracting and/or calculating at least one second quantification image of the spatial distribution of flow of said fluid in said body at said stress condition, said at least one second quantification image being defined by pixels or group of pixels values having an appearance scale univocally correlated to a parameter representative of the perfusion at said stress condition;
- combining the two quantification images to obtain at least an image defined by pixels or group of pixels values having an appearance scale univocally correlated to a combination of corresponding pixels or group of pixels of the two quantification images.

2. Method according to claim 1, wherein the two quantification images are aligned so that pixel values of one image can be compared to homologous pixel values of the other image.

3. Method according to claim 2, wherein the step of aligning the images comprises deforming one or both images to allow cross reference points identified on both images to overlap.

4. Method according to claim 2 or 3, wherein images are aligned manually or automatically using optimal-likelihood-based processing.

5. Method according to any preceding claim, wherein the two quantification images are combined through a non-linear software and/or hardware device, such as a divider and/or a multiplier and/or a logarithmic and/or a cross-correlation circuit or the like.

6. Method according to any preceding claim, wherein the step of combining the two quantification images comprises calculating the ratio of pixel values of the second image to corresponding pixel values of the first image or vice versa to obtain an image where some or all the pixels have values corresponding to such ratio.

7. Method according to any preceding claim, wherein one or more thresholds are defined to determine which pixels of the generated image(s) are a combination of the corresponding pixels of the first and second image and/or which pixels of the generated image(s) correspond to pixels related to only one of the two images and/or are filtered out.

8. Method according to claim 7, wherein values below such threshold(s) are neglected and/or corrected to filter noise in the generated image.

9. Method according to any preceding claim, wherein the step of extracting and/or calculating at least one quantification image from each sequence of images comprises one or more steps selected from the group consisting of:
- extracting the last frame of the sequence;
- extracting a frame of the sequence at an intermediate time;
- extracting/calculating the image of the sequence having the maximum brightness;
- determining a parametric image.

10. Method according to claim 9, wherein the step of determining a parametric image comprises:
- defining an evaluation function for each sequence of images, said evaluation function having at least one parameter;
- calculating for each pixel or group of pixels of each sequence the value of said parameter for best fitting said evaluation function with a curve representing the values of said pixels or group of pixels obtained from such sequence of images;
- constructing a parametric image for each sequence of images by defining a pixel appearance scale univocally correlated to said at least one parameter.

11. Method according to claim 10, wherein said estimation function is of the form y(t)=A(1-e^{-Bt}), where y(t) is the pixel value depending from time, t is the time at which the pixel value has been determined in the image and A and B are parameters giving the best fit of said estimation function.

12. Method according to claim 11, wherein said parameters are imaged in a two-dimensional or a three-dimensional image, or in any known representation of a function of one, two, or more variables.

13. Method according to any preceding claim, wherein the pixel values comprise the brightness of black and white digital images or one or more variables of colour digital images like hue, saturation, colour or the like.

14. Method according to any preceding claim, wherein more quantification images are extracted and/or calculated from each sequence of images, more images being generated by combining corresponding images at rest and stress condition.

15. Method according any preceding claim, wherein one image is generated, such image being the ratio of two images extracted/calculated from a combination of images extracted and/or calculated respectively from the sequence of images at stress and from the sequence of images at rest.

16. Method according to any preceding claim, wherein the generated image(s) is/are displayed overlaid with the image(s) of the first and/or the second sequence of images and/or with the corresponding quantification image(s).

17. A method according to one or more of the preceding claims wherein the sequences of images are echographic or MRI or SPECT or PET or X-Ray images or the like.

18. A method according to one or more of the preceding claims wherein the images of the sequences are obtained with one or more imaging modes selected from the group consisting of: Doppler, power Doppler, B-mode, Harmonic imaging, Contrast imaging.

19. A method according to one or more of the preceding claims wherein the permeable body is a biological tissue, the sequences of images being representative of the spatial distribution of blood flow in such tissue.

20. A method according to claim 19, wherein the permeable body is the heart, the sequences of images being representative of the blood perfusion in the myocardium.

21. A method according to claim 20, wherein the pixels of the images of the sequences have brightness values related to the concentration of a contrast media perfusing the myocardium at the time the images are taken.

22. A method according to claim 20 or 21, wherein, for each sequence of images representative of a condition of the heart, a perfusion image is determined, such perfusion image giving a synthetic spatial representation of the perfusion process of at least part of the myocardium at such condition.

23. A method according to claim 22, wherein one or more differential perfusion images representative of the capacity of the myocardium to react to a demand for an increased blood flow are determined by combining perfusion images obtained from sequences of images of the myocardium at rest and hyperemic condition.
